# EUROPEAN PATENT SPECIFICATION

(11) **EP 1 415 158 B1**
(45) Date of publication and mention of the grant of the patent: **15.06.2005**
(21) Application number: 02762376.8
(22) Date of filing: 19.07.2002
(51) Int. Cl.: G01N 33/569

(54) **METHOD FOR SELECTING ANTIBODY EXPRESSING CELLS**
VERFAHREN ZUR SELEKTION VON ZELLEN DIE ANTIKÖRPER EXPRIMIEREN
PROCEDE POUR SELECTIONNER LES CELLULES EXPRIMANT DES ANTICORPS

(30) Priority: 27.07.2001 GB 0118337
(43) Date of publication of application: 06.05.2004
(73) Proprietor: Lonza Group AG, 4052 Basel (CH)
(72) Inventor: RACHER, Andy, Reading RG7 4UY (GB); SINGH, Rabinder, West Midlands B75 5TF (GB)
(86) International application number: PCT/EP2002/008054
(87) International publication number: WO 2003/012449

(56) References cited:
- WO-A-99/58977
- HOLMES PAUL ET AL: "Application of flow cytometry to bioprocess development, validation and control." CYTOMETRY SUPPLEMENT, [Online] no. 10, 2000, page 53 XP002250981 The XX Congress of the International Society for Analytical Cytology;Montpellier, France; May 20-25, 2000 ISSN: 1046-7386 Retrieved from the Internet: <URL:http://www.wiley.com/legacy/pro...ife /cytometry/isac2000/6133.htm > [retrieved on 2003-08-07]
- FRYKMAN SCOTT ET AL: "Quantitating secretion rates of individual cells: Design of secretion assays." BIOTECHNOLOGY AND BIOENGINEERING, vol. 59, no. 2, 20 July 1998 (1998-07-20), pages 214-226, XP002250982 ISSN: 0006-3592
- HOLMES PAUL ET AL: "Improved cell line development by a high throughput affinity capture surface display technique to select for high secretors." JOURNAL OF IMMUNOLOGICAL METHODS, vol. 230, no. 1-2, 19 November 1999 (1999-11-19), pages 141-147, XP002250983 ISSN: 0022-1759 cited in the application

## Description

The present invention relates to the field of biotechnological production of. It is an object of the present invention to devise a method for selecting antibody producing cells and for assessing cell line stability.

Selection of antibody producing cells versus non-producing cells and in particular the selection of high producing cell lines is an important first step in the development of any bioprocess. For protein production from animal cells, such selected cell lines have traditionally been isolated by rounds of limiting dilution cloning followed by product analysis. This traditional process is time consuming and costly. Two rounds of cloning are required to improve the theoretical confidence of achieving clonality. Clonality is important to avoid overgrowth of high producers by low productivity variants which usually have higher growth rates than high producers. In total, the traditional process can take in excess of 8 months to complete. Furthermore, practical considerations may limit the number of cells actually screened and thus may render the detection of low abundance high-producing cell clones a mere question of chance.

Assessment of cell line stability during the development process can further require analytical cloning, which can take between 6 to 7 weeks. In addition, analytical cloning involves assessment of 200-300 clones, compared to the analysis of many thousands that is possible with flow cytometry, which therefore provides a more detailed and accurate picture regarding the size of the non-producing population. Previously reported secretion assays have not been of sufficient sensitivity to allow resolution and thus quantification of distinct sub-populations.

Flow cytometry (FC) has also made it possible to monitor productivity of a very large number of cells and to isolate suitable single cells. FC requires labelling of cells with fluorophor, whose fluorescence is quantified in FC and should correlate with the desired property. However, the amount of antibody displayed membrane-bound on the surface of a cell line does not correlate well with its secretion rate for soluble antibody (Meilhoc et al., Application of flow cytometric measurement of surface IgG in kinetic analysis of monoclonal antibody synthesis and secretion by murine hybridoma ccell-line in low serum and serum-free media, Hybridoma 9, 67-175). Therefore, simply staining the displayed antibody with a detection antibody has not proven effective for isolating highly productive clones. Two fundamentally different approaches have been chosen to overcome this problem and to directly select for high producer cells of secreted antibodies.

In a the first method, single cells and concomittant secreted antibody are encapsulated in gel droplets. The entire droplet is then subjected to labelling and is sorted by means of FC. This approach suffers from the disadvantage that it is user unfriendly, that in order to ensure single cell occupancy of gel droplets, only about 5% of the gel droplets created comprise a cell whilst about 95% of the gel particles analyzed are devoid of cells. In addition, encapsulation/decapsulation can reduce the viability of some cell types commonly used for expression of recombinant protein, most notably meyeloma NSO cells.

In the second approach, secreted antibodies are retained by artificially created affinity sites or receptors on the cell surface in order to assess the amount of secreted antibodies subsequently by FC. Holmes et al. (J. of Immunol. methods, 230 (1999), 141-147) describe an assay involving the biotinylation of plasma membrane proteins at room temperature. The biotin promotes further binding first of avidin and secondly of a biotinylated capture antibody. The capture antibody recognizes the secreted antibody from a NSO cell line and anchors it to the cell surface. Fluorescent detection and cell sorting of low abundance high producer cell clones by FC is then achieved by incubating the cells with the captured secreted antibody with a third, fluorecently labelled detection antibody. Addition of a viscosity agent such as gelatine to the incubation media prevents cross-talk between individual clones (i.e. diffusion and binding of secreted antibody from producer cells to non- or low-producer cells).

The main disadvantage of the published method is that it provide relatively low sensitivity as to discriminate for high and low level producer cells and to allow for precise sorting of low abundance high producer cells. Using the published method, it was not possible to detect the binding of a secreted chimeric antibody from a given cell line to the cell surface via a capture antibody. Furthermore, the capture antibody necessarily stems from mammalian cell culture, which may include fetal serum, and will often be stabilized with bovine serum albumin as a commercial antibody preparation. Use of capture antibody therefore entails the risk of viral or BSE contaminations of the clonal cell cultures.

It is the object of the present invention to devise another method to efficiently detect and select suitable antibody expressing single cells from a pool of cell clones. This object is achieved by the method according to independent claim 1.

Possible embodiments of the invention are shown in the figures. It is shown in
Fig.1 a: schematic drawing of the capture and detection principle for assaying for secreted antibody and b: comparison of binding capacity for capture antibody and Protein A
Fig.2 Fluorescent cytometry (FC) analysis of samples from secretion assay of the invention
Fig.3 Flurorescent cytometry (FC) analysis of samples from secretion assay comparing effect of protein A to anti-human IgG antibody for capture of secreted antibody.

The method according to the present invention for selecting cells secreting a first antibody comprises the steps of
a) linking an avidin to a cell surface of a cell
b) incubating the cell with a biotinylated polypeptide comprising at least one functional antibody binding domain from protein G or from protein A
c) incubating the cell with a second antibody that is fluorescently labelled and that recognizes and binds an epitope on the first antibody and rinsing off unbound second antibody after incubation
d) selecting fluorescently labelled cells that have bound an amount of second antibody, preferably and expediently by means of fluorescent analysis flow cytometry (FC). FC allows concomittant sorting of fluorescently labeled cells.

The producer cell secreting the antibody may be any cell employed for secreting antibodies such as e.g. hybridoma, myeloma or CHO cells. More preferably, the producer cell is a myeloma cell line, most preferably, it is an NSO cell line.

Consequently, the antibody may be any naturally occuring or engineered antibody or antibody fragment such as chimeric or monovalent or bispecific antibodies, including the more recently known single heavy chain antibodies of camels and llamas (Trends Biochem. Scien. (2001), 26, 230). Preferably, such antibodies according to the present invention always comprise binding sites for high affinity binding of bacterial protein G or protein A as comprised in the naturally occuring Fc portion of Immunoglobulin (Ig) of various classes (IgG, IgA, IgE, IgM). Preferably, the antibody according to the present invention comprises an Fc portion of an IgG. Most preferably, the antibody according to the present invention is an IgG or comprises at least one heavy chain of IgG.

The avidin according to the present invention is conventional avian avidin (approx. 67 kD) or any other functional i.e. biotin-binding homologue thereof such as e.g. Streptavidin (60 kD) from Streptomyces species or any chemically or genetically engineered variant of such naturally occuring avidins. An example for such engineered variant is Neutravidin (Pierce, Rockford/IL) which is deglycosylated avidine devoid of carbohydrate, having an pI between 6-7 and being further engineered as not to comprise the tripeptide RYD domain comprised in Streptavidin that may mediate non-specific binding to cell surface receptors. Preferably, the avidin according to the present invention is avian avidin, deglycosylated avian avidine or is a neutravidine. Most preferably, the avidine according to the present invention is a neutravidine. Such neutravidine shows maximum capacity for biotin binding which capacity is approximately in the range of 14 µg biotin/mg of protein.

The avidin can be linked to the cell surface e.g. by crosslinking. Conventional bifunctional crosslinking reagents well-known in the art can be employed which comprise reactive groups such as oxiranes, aldehydes, succine imides or photo-reactive compounds such as N-[N-4-azido-tretrafluorobenzoyl)biocytinyloxy]succinimide, SAND (sulfosuccinimidyl 2-[m-azido-o-nitrobenzamido]-ethyl-1,3-dithiopropionate). It goes without saying that the crosslinking does occur either with reactive groups of lipids (e.g. hydroxyfunction of phoshatidylserine) or, more likely, with the reactive groups displayed on the surface of membrane proteins.

In a prefered embodiment, the avidin is attached non-covalently onto the cell surface after having linked a biotin moiety covalenty, by means of a monofunctional crosslinking reagent carrying a biotin label, to the cell surface. The avidin recognizes and binds to the biotin moiety on the cell surface. More preferably, such biotinylation is carried out at a temperature below 10°C, most preferably at about 4°C or below. At this temperature, efficiency ofbiotinylation increases with time. Expediently, the reaction time for biotinylation of the cell surface is in the range of 30-60 min..

If avidin is to be captured to the cell surface by means of a biotin label on the cell surface, it is further prefered that the cells are incubated in a solution comprising at least 50 µg/ml avidin, more preferably at least 100 µg/ml avidin, the term 'avidin' refering to the avidin according to the present invention. Avidin molecules usually have about 4 binding sites for biotin. If a biotin label is employed for coating the cell surface and avidin is subsequently incubated in excess of the covalently linked biotin label on the cell surface, one biotin label will bind to one avidin molecule leaving open about 3 further binding sites for biotin labels.

Further prefered is that the biotin moiety that is covalently linked to the cell surface and which functions to anchor avidin non-covalently to the cell surface is linked to the cell surface by means of a spacer moiety, e.g. a linear alkyl chain, which extends for at least 10 Å, more preferably at least 20 Å, most preferably at least 30 Å.

In the next step of the method according to the present invention, a biotinylated polypeptide encompassing at least one antibody binding domain is incubated with the avidin labelled cells and becomes bound to the avidin that is now linked to the cell surface. The antibody binding domain according to the present invention is not derived from the the antigen-binding or complementarity determining portion of an immunoglobuline or immunoglobuline fragment. It is a non-immunoglobuline-derived antibody binding polypeptide. The polypeptide may be e.g. protein A (Surolia, A. et al., 1982, Protein A: Nature's universal antibody, TIBS 7, 74-76; Langone, J., 1982, Protein A of staphylococcus aureus and related immunoglobulin receptors, Adv. in Immunology, 32: p.156-241), protein G or protein L. Protein L is from *Peptostreptococcus* species and has the unique ability to bind through kappy light chain interactions without interfering with an antibody's antigen-binding site (Kastem, W. et al., 1992, Structure of protein L and identification of a repeated immunoglobulin light-chain binding domain, J. Biol. chem. 267,12820-12825), thus binding to all classes of Ig as well as to single chain variable fragments (ScFv) and Fab fragments.

According to the present invention, the use of such antibody-binding domain comprising polypeptide leads to enhanced sensitivity in a secretion assay according to the present invention, allowing to sufficiently discriminate high producer cell clones from low or non-producers. The secretion assay according to the present invention also allows for easy and fast assessment of cell line stability. Assessment of cell line stability during a cell line development process traditionally required analytical cloning of at least 200-300 clones, which can take between 6 to 7 weeks. In conjunction with flow cytometry, the assay which is the subject of this application is sensitive enough to resolve and quantify low or non-producing sub-populations of thousands of cells, and thereby give information on cell line stability in only 5 hours.

Preferably the antibody binding domain is from protein A or protein G. Protein A of *Staphylococcus* species and protein G of *Streptococcus* species are well-known protein agents that specifically bind to the Fc portion of antibodies (Boyle, M. , Bacterial Immunoglobulin-Binding proteins, Academic Press, Sand Diego 1990). They have found widespread application in biotechnology for affinity purification of IgG of almost any animal species or subclass. Staphylococcal protein A binds to a similiar site on the Fc fragment of IgG as does streptococcal protein G, involving in both cases e.g. in human IgG-Fc residues 252-254, 433-435 and 311, as shown in Deisenhofer et al. (1981, Biochemistry 20; 2361-2370) and in Sauer-Eriksson et al. (1995, Structure 3, 265-278).

Furthermore, WO 00/7428 describes an isolated antibody binding B 1 domain polypeptide of bacterial protein G which binds a Fab fragment of an IgG but substantially does not bind an Fc fragment of an IgG. The use of this Bl domain is another prefered embodiment of the present invention.

In general, antibody domains according to the present invention may be used as isolated domains or as fusion proteins with other polypeptide moieties. It is also possible to fuse several antibody binding domains of e.g. proteins A, G, L, or of still functional variants thereof that have been artificially engineered by amino acid substitution, deletion or insertion. It is also possible to employ variants of e.g. proteins A, G, L, etc., respectively, that carry deletions or are truncated.

Preferably, the polypeptide comprises no or only 1-2 sites of glycosylation, more preferably it lacks any carbohydrate.

Preferably, a polypeptide comprising at least four antibody binding sites, most preferably intact protein A of about 42 kD molecular weight is employed in the present invention. Such intact protein A has 4-5 high affinity binding sites for the Fc portions of IgG. In conjunction with the multivalency of avidin for biotin moieties, this leads to considerable amplification of the amount of secreted antibody bound and thus to signal amplification.

Biotinylation of the polypeptide may be achieved by any of the methods described above for biotinylation of the cell surface. In addition, it is also and conveniently possible to conjugate a biotin moiety to tyramine, whose covalent linking to the polypeptide is catalyzed by oxygen free radicals generated by hydrogen peroxidase in the presence of hydrogen peroxide.

It is also possible, and this being a further object of the present invention, to employ directly an polypeptide-avidine conjugate which polypeptide carries at least one antibody binding domain of protein A or protein G. Crosslinking of polypeptide and protein moieties, respectively, is well-known in the biochemical arts and may be achieved with a multitude of reagents (Fasold et al., bifunctional reagents for the crosslinking of proteins, Angew. Chem. Int. Ed. Engl. (1971), 10: 795-801). For instance, coupling to amino groups of the amino acid lysines by means of N-hydroxysuccinimide (NHS) esters is well-known in the art. Prefered embodiments and definitions of the present invention apply likewise to this object.

Preferably, the biotin moiety is linked to the polypeptide by means of a spacer moiety, which spacer arm has a length of at least 15 Å, more preferably of at least 22 Å, most preferably of at least 30 Å. Suitable spacer arms can be e.g. linear alkyl moieties. Such extended spacer arms reduce steric hindrance when binding several biotinylated molecules to one avidin complex.

Further prefered is that the antibody binding domain comprising polypeptide according to the present invention carries at least 3 biotin moieties covalently linked to the polypeptide or protein, more preferably at least 6 biotin moities and most preferably 6-10 biotin molecules per polypeptide or protein, respectively. The extend of biotinylation of the polypeptide is easily governed by the number of reactive groups in the polypeptide (e.g. amino groups of lysine, sulhydrylgroups of cysteine) and the proportion of biotinylation reagent to polypeptide upon biotinylation.

The second, detection antibody according to the present invention may be any conventional, fluorescently labelled antibody or antibody fragment that specifically binds to the secreted first antibody whose expression by a cell clone is to be monitored by the method of the present invention. In the scope of the present invention, 'detection antibody' shall also be construed as to comprise any suitable combination of a first and second detection antibody, only the latter being fluorescently labelled, as is conventionally employed in related techniques such as e.g. Elisa techniques. Equally, a fluorescently labelled antigen recognized by the first, secreted antibody, may be employed for detection. Expediently, in between steps b and c of the present invention, a short intervening incubation period in cell culture medium allows to saturate artifically created antibody binding sites on the cell surface with secreted antibody.

Preferably, during or at latest after incubation of the cells with the biotinylated polypeptide according to the method of the present invention, the cells are cultured in a medium comprising a viscosity increasing agent. This measure prevents cross-feeding between cells by artificially elevating the viscosity of the medium. Such agent can be e.g. methyl cellulose, PEG, starch, polysaccharides from algae, bacteria or plants, or gelatine. In a prefered embodiment, gelatine in a concentration of at least 10% (w/w) is employed.

In a further, particularly preferred further embodiment, during or at latest after incubation of the cells with the biotinylated polypeptide and prior to staining the cells with the fluorescently labelled antibody or antigen according to the method of the present invention, the cells are cultured in a medium comprising non-biotinylated protein A, or another non-capture, competing antibody binding domain comprising polypeptide according to the present invention, in a concentration of at least 0.5 µg/mL, more preferably in a concentration of at least 4 µg/mL, most preferably in a concentration of at least 8 µg/mL. Expediently, such non-capture, competing protein A or the like is used in combination with a viscosity increasing agent as described above, in order to prevents cross-feeding between cells.

Preferably, prior to staining the cells with the fluorescently labelled antibody or antigen according to the method of the present invention and after having allowed secreted antibody to bind to the biotinylated polypeptide that was immobilized on the cell surface, a third, blocking antibody is added to the cells, e.g. in a concentration of at least 0.5 to 5 mg/ml. Such third blocking antibody is able to bind to the remaining antibody binding sites of the biotinylated polypeptide but is not recognized and bound, respectively, by the second fluorescently labelled antigen or antibody employed for detection. This measure further contributes to eliminate late cross-feeding between clones and thus to improve sensitivity of and discrimination by the assay of the present invention. It goes without saying that such measure can be combined in any way with the other proposed measures, e.g. the viscosity increasing agent and/or the competing non-biotinylated protein A and the like, in order to synergistically enhance discrimination and thus sensitivity of the assay.

### Examples

In all experiments, cells were taken from exponential growth phase of batch culture with viability being greater than 95% as assayed by standard dyed-cell counting techniques.

### 1. Comparative experiment

Cell line 6A1(100)3 (obtained from Lonza Biologies) derived from NSO myeloma cell line was used. It is a GS-transfectant cell line (Bebbington, C. et al., 1992, High-level expression of a recombinant antibody from myeloma cells using a glutamine synthetase (GS) gene as an amplifiable selectable marker, Bio/Technology 10:169-175) that is secreting human chimeric IgG antibody cB72.3 specific to the the breast tumour antigen TAG73. The cells were treated exactly as described in the publication Holmes et al.(ibd.). The result was disappointing, with no reproducible, significant difference in fluorescence between the secreting cells and an untreated negative control cell line (NSO cell line ECACC No. 85110503) being observable. The experiment was repeated with a non-producing GS transfected myeloma cell line and with human IgG added externally to the cells, and again, there was no appreciable difference in mean fluorescence.

### 2. Secretion assay

### 2.1 Capture of secreted antibody

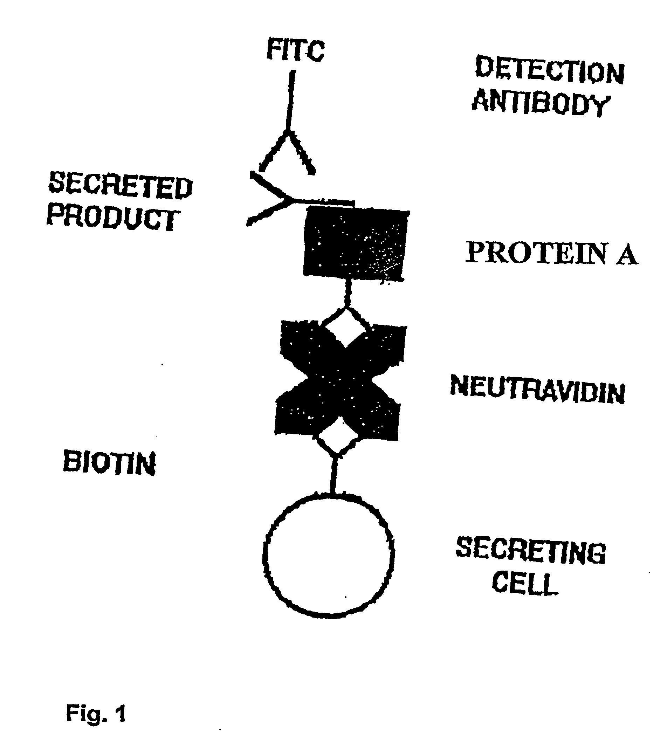

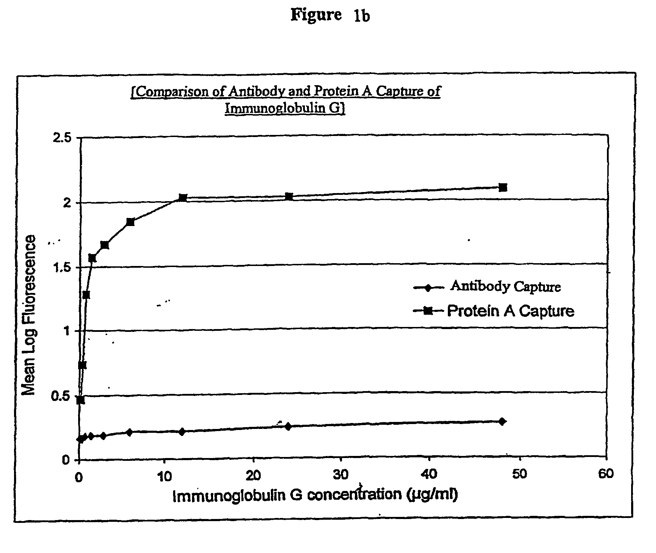

The principle of capture and detection of secreted antibody is schematically depicted in Fig. 1. The cell line 6A1(100)3 secreting chimeric antibody cB72.3 was again chosen as an experimental model. The secretion matrix was constructed as follows. 10⁷ 6A1(100)3 cells were washed in 25 mL of pH8 PBS and re-suspended in 1 mL of 1 mg/mL NHS-LC-biotin (catalogue no. 21336, succinimidyl-6-(biotinamido) hexanoate; Pierce, Rockford, IL/U.S.A.) in physiological, standard PBS (pH8). Following 40 minutes incubation at 4°C, the cells were washed twice in PBS (pH7), and re-suspended in 1 mL of PBS (pH7). 128 µL of a 1 mg/mL neutravidin™ (Pierce, UK) solution was added, and the cells incubated at room temperature for 15 minutes followed by a further wash with 50 mL of PBS (pH7). Following re-suspension in 1 mL of pH 7 PBS, non-biotinylated protein A was added to a final concentration of 1 µg/mL: the mixture was incubated at room temperature for 5 minutes. Next, 52 µL of a 1 mg/mL biotinylated Protein A (Pierce, Rockford, IL/U.S.A.; carries 6-10 biotin labels per protein) solution was added and incubated for a further 15 minutes at room temperature. The concentration of biotinylated Protein A applied for incubation was not yet saturating. Independent experiments confirmed that even at a concentration of 120 µg/ml biotinylated Protein A, binding was not approaching saturation (data not shown).

Samples to be used as positive control were then dosed with the appropriate volume of purified cB72.3 IgG to give a concentration of 6.6 µg/mL.

Samples to be used as negative control consisted of cells being devoid of a biotin/avidin/capture protein A affinity matrix. These samples were processed as all other samples starting with incubation in the secretion medium.

All samples that were to be used in the full secretion assay were exposed to the following procedure: Cells were resuspended in 10 mL of secretion medium (cell culture medium as described in Holmes, P. et al., Improved cell line development by a high throughput affinity capture surface display technique to select for high secretors, J. of Immunological methods, 230 (1999), 141-147 for culture of NSO 6A1(100)-3 cell line enriched with 10% gelatine). This medium also contained 8 µg/mL of non-biotinylated protein A (Sigma, UK) in order to capture antibody that diffuses away from producer cells and thereby prevent it from binding to non-producer cells. The cells were incubated in the secretion medium for 15 minutes at 4 °C.

### 2.2 Fluorescence Labelling

Bound secreted antibody was detected using mouse anti-human kappa light chain FITC conjugate (Sigma, UK) at a final dilution of 1/500.
Cells treated as described under 2.1 were washed once in 25 ml PBS (pH 7) and 320 µl of a 1mg/ml *murine* IgG blocking antibody (Sigma, UK) was added and mixed with the cell pellet and incubated for 5 minutes. The cells were then washed for the second time with 25 mL of PBS (pH7) and resuspended in 1 ml of PBS. 5 µL of mouse *anti-human* Kappa light chain FITC detection antibody (Sigma, UK) was added, and the cells incubated for 15 minutes. Following a further wash in 25 ml PBS, the cells were resuspended in 1 mL PBS for analysis by flow cytometry.

### 2.3 Flow cytometry

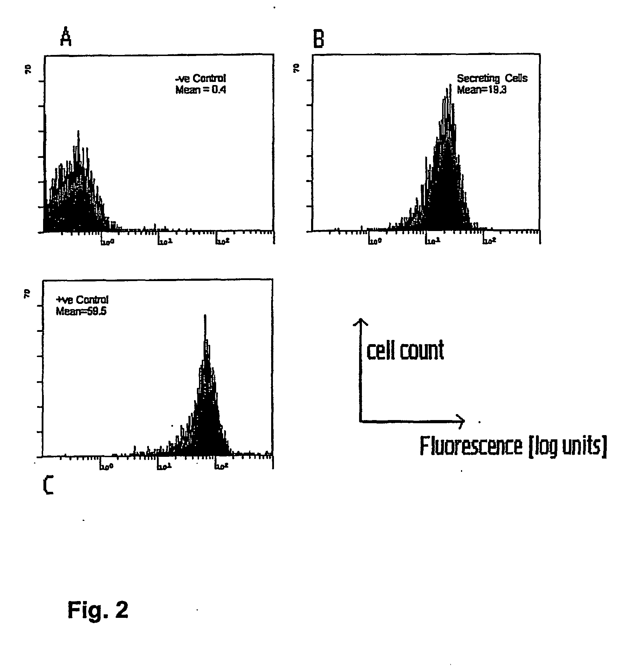

After staining as described in the previous section, cells were analysed using a Coulter Epics Elite flow Flow Cytometer with 488 nm and 515 nm excitation and detection wavelengths respectively. Log side scatter vs. forward scatter was used to identify the viable population which was gated and the level of fluorescence (and therefore the amount of bound antibody) was measured for this population. Distributions for (A) negative control (i.e. without affinity matrix), (B) cells with secreted antibody captured by the affinity matrix and (C) positive control cells incubated with externally added, purified IgG captured by the affinity matrix are all shown in Fig. 2 as cell count vs. fluorescence intensity. Secreting cells B exhibited a mean fluroescence of 19.3 which was a 50-fold increase in mean fluorescence as compared to the negative control A. The positive control C showed a mean of 59.5 and thus a 125-fold increase over the negative control. Numeric values of mean fluorescence according to Fig. 2 are given in table 1

**Table 1**

| Sample | Mean fluorescence [arbitrary units] |
|---|---|
| A: negative control | 0.4 |
| B: secreting cells | 19.3 |
| C: positive control (+ exogenous IgG) | 59.5 |

### 3. Comparative Example

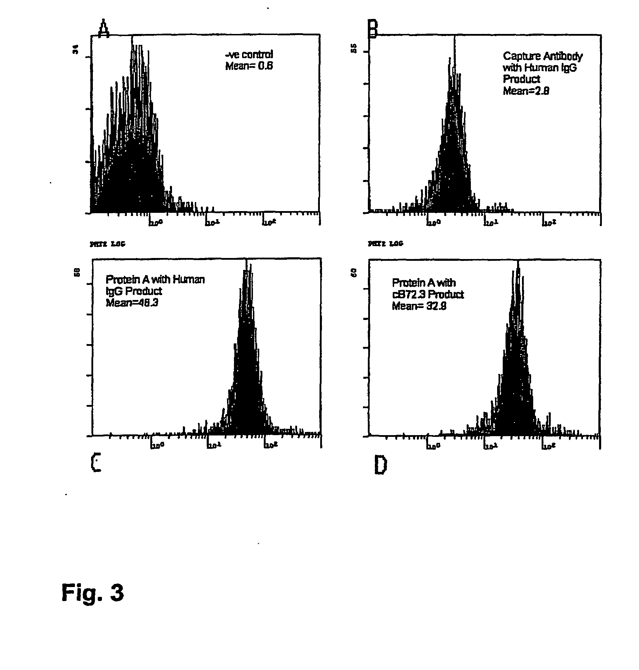

The effectiveness of biotinylated capture antibody binding of secreted antibody at the cell surface was compared with that of biotinylated protein A. In the comparative experiment, the secretion assay was performed exactly as described in section 2 with the exception that PBS devoid of any viscosity increasing agent was used for incubation instead of secretion medium for all samples. Apart from standard negative control (A), positive control (C) and cB72.3 secreting cells (D) as described in the previous sections, one additional sample (B) was prepared by substituing biotinylated protein A with a biotinylated mouse anti-human IgG Fc-specfic capture antibody (Sigma, UK) which was immobilised on the cell surface by incubation at 50 µg/ml for 15 minutes, this concentration being close to saturation and being comparable to the amount of protein A used in the previous experiments. Sample (B) was subsequently incubated with exogenously added human IgG and further processed as has been described for the positive control above. Distributions for all samples are shown in Fig. 3 as cell count vs. fluorescence intensity after flow cytometry fluorescence analysis, as was used in Fig. 2 (Fig. 3: A: negative control, B: capture antibody, C: positive control, protein A, D: secreted cB72.3, protein A). Numeric mean fluorescence values according to the distributions given in Fig. 3 are listed in table 2. The use of capture antibody with human IgG model product (sample B) gave a 5-fold increase in mean flurorescence as compared to the negative control cells. However, when protein A was used (sample C), there was an 80-fold increase in mean fluorescence, thus demonstrating that protein A was more effective than capture antibody. Protein A was also highly effective when the technique was used to capture and detect secreted cB72.3, with an approximately 55-fold increase in mean fluorescence as compared to the negative control.

Samples prepared exactly as described in Holmes et al. (ibd.) showed no increase over the negative control (data not shown).

**Table 2**

| Sample | mean fluorescence [arbitrary units] |
|---|---|
| A: Negative control | 0.6 |
| B: Capture Antibody + exogenous IgG | 2.8 |
| C: Capture protein A + exogenous IgG | 46.3 |
| D: Capture protein A + secreted cB72.3 | 32.9 |

Comparing the data of experiments B vs. C,D, it is clear that substitution of capture antibody by protein A gives drastic improvements in sensitivity of detection and capacity of binding, respectively. Theoretically, protein A should have an excess of two binding sites (total: 4) as compared to capture antibody, and thus might allow to double the amount of secreted antibody bound (theoretical 2-fold intensity enhancement). This expectation would have constituted a theoretical maximum, since a high-affinity binding antibody can exceed the binding affinity of protein A by about one order. In contrast, the actually observed intensity enhancement by protein A vs. capture antibody of about 15-20-fold largely exceeds the theoretically predicted value, surprisingly and for unknown reason.

Fig. 1 b shows comparison of binding capacity for capture antibody binding Fc portion of exogenously added IgG vs. protein A binding such exogenous IgG. Samples were essentially prepared as described in present parts of this section, except that both the amount of capture antibody and protein A, respectively, used for incubation was doubled in order to ensure saturating binding. Purified IgG was added to defined volume of cell pellet, in order to ensure precisely predetermined concentration of exogenous IgG during incubation.

## Claims

1. Method of selecting cells secreting a first antibody, comprising the steps of
a) linking avidin to a cell surface of a cell
b) incubating the cell with a biotinylated polypeptide comprising at least one functional antibody binding domain of protein A, protein G, protein L or functional variants thereof.
c) incubating the cell with a second antibody or antigen that is fluorescently labelled and that recognizes and binds an epitope on the first antibody
d) selecting fluorescently labelled cells that have bound an amount of second antibody, by means of fluorescent analysis flow cytometry.

2. Method of claim 1, **characterized in that** the biotinylated polypeptide is a protein G or a protein A.

3. Method of claim 1, **characterized in that** the avidin is bound to the cell surface after covalent biotinylation of the cell surface has been carried out.

4. Method of claim 1, **characterized in that** during or at latest after incubation with the biotinylated polypeptide, the cells are cultured in a medium comprising a viscosity increasing agent.

5. Method of selecting cells secreting a first antibody, comprising the steps of
1) linking avidin to a cell surface of a cell wherein the avidin is conjugated to a polypeptide comprising at least one functional antibody binding domain from protein G or from protein A.
2) incubating the cell with a second antibody or antigen that is fluorescently labelled and that recognizes and binds an epitope on the first antibody
3) selecting fluorescently labelled cells that have bound an amount of second antibody, by means of fluorescent analysis flow cytometry.

6. Method according to claim 5, **characterized in that** the avidin is bound to the cell surface after covalent biotinylation of the cell surface has been carried out.

7. Method according to one of the preceding claims, **characterized in that** the cell is an antibody-secreting cell.

8. Method according to claim 7, **characterized in that** the cell is a hybridoma, myeloma or CHO cell.

9. Method according to one of the preceding claims, **characterized in that** the antibody comprises binding sites for high affinity binding of bacterial protein G or A.

10. Method according to one of the preceding claims, **characterized in that** the antibody is an IgG or comprises an Fc portion of an IgG.

11. Method according to one of the preceding claims, **characterized in that** the avidin is neutravidine.

12. Method according to claim 1, **characterized in that** the biotin moiety is linked to the polypeptide by means of a spacer moiety which spacer moiety has a length of at least 15 Å.

13. Method according to claim 1 or 12, **characterized in that** the biotinylated polypeptide carries at least 3 biotin moieties covalently linked to the polypeptide.

14. Method according to claim 13, **characterized in that** the biotinylated polypeptide comprises 6-10 biotin moieties.

15. Method according to claim 3 or 5, **characterized in that** biotinylation of the cell surface has been carried out at a temperature of less than 10oC.

## Patentansprüche

1. Verfahren zur Selektion von einen ersten Antikörper sezernierenden Zellen, bei dem man
a) Avidin mit einer Zelloberfläche einer Zelle verknüpft,
b) die Zelle mit einem biotinylierten Polypeptid, das wenigstens eine funktionelle Antikörperbindungsdomäne aus Protein A, Protein G, Protein L oder aus einer funktionelle Variante davon umfaßt, inkubiert,
c) die Zelle mit einem zweiten Antikörper oder Antigen, der bzw. das fluoreszenzmarkiert ist und ein Epitop auf dem ersten Antikörper erkennt und bindet, inkubiert,
d) fluoreszenzmarkierte Zellen, die eine Menge an zweitem Antikörper gebunden haben, selektioniert, mittels Fluoreszenzanalyse-Durchflußzytometrie.

2. Verfahren nach Anspruch 1, **dadurch gekennzeichnet, daß** es sich bei dem biotinylierten Polypeptid um ein Protein G oder ein Protein A handelt.

3. Verfahren nach Anspruch 1, **dadurch gekennzeichnet, daß** das Avidin an die Zelloberfläche gebunden wird, nachdem eine kovalente Biotinylierung der Zelloberfläche durchgeführt wurde.

4. Verfahren nach Anspruch 1, **dadurch gekennzeichnet, daß** die Zellen während oder spätestens nach der Inkubation mit dem biotinylierten Polypeptid in einem Medium mit einem viskositätssteigernden Mittel kultiviert werden.

5. Verfahren zur Selektion von einen ersten Antikörper sezernierenden Zellen, bei dem man
1) Avidin mit einer Zelloberfläche einer Zelle verknüpft, wobei das Avidin an ein Polypeptid mit wenigstens einer funktionellen Antikörperbindungsdomäne aus Protein G oder aus Protein A konjugiert wird,
2) die Zelle mit einem zweiten Antikörper oder Antigen, der bzw. das fluoreszenzmarkiert ist und ein Epitop auf dem ersten Antikörper erkennt und bindet, inkubiert,
3) fluoreszenzmarkierte Zellen, die eine Menge an zweitem Antikörper gebunden haben, selektioniert, mittels Fluoreszenzanalyse-Durchflußzytometrie.

6. Verfahren gemäß Anspruch 5, **dadurch gekennzeichnet, daß** das Avidin an die Zelloberfläche gebunden wird, nachdem eine kovalente Biotinylierung der Zelloberfläche durchgeführt wurde.

7. Verfahren gemäß einem der vorhergehenden Ansprüche, **dadurch gekennzeichnet, daß** es sich bei der Zelle um eine Antikörper sezernierende Zelle handelt.

8. Verfahren gemäß Anspruch 7, **dadurch gekennzeichnet, daß** es sich bei der Zelle um eine Hybridom-, eine Myelom- oder eine CHO-Zelle handelt.

9. Verfahren gemäß einem der vorhergehenden Ansprüche, **dadurch gekennzeichnet, daß** der Antikörper Bindungsstellen zur hochaffinen Bindung von bakteriellem Protein G oder A enthält.

10. Verfahren gemäß einem der vorhergehenden Ansprüche, **dadurch gekennzeichnet, daß** es sich bei dem Antikörper um IgG handelt oder daß der Antikörper einen Fc-Anteil eines IgG enthält.

11. Verfahren gemäß einem der vorhergehenden Ansprüche, **dadurch gekennzeichnet, daß** es sich bei dem Avidin um Neutravidin handelt.

12. Verfahren gemäß Anspruch 1, **dadurch gekennzeichnet, daß** die Biotingruppierung mit dem Polypeptid mittels einer Spacer-Gruppierung verknüpft ist, wobei die Spacer-Gruppierung eine Länge von mindestens 15 Å aufweist.

13. Verfahren gemäß Anspruch 1 oder 12, **dadurch gekennzeichnet, daß** das biotinylierte Polypeptid wenigstens 3 mit dem Polypeptid kovalent verknüpfte Biotingruppierungen trägt.

14. Verfahren gemäß Anspruch 13, **dadurch gekennzeichnet, daß** das biotinylierte Polypeptid 6-10 Biotingruppierungen enthält.

15. Verfahren gemäß Anspruch 3 oder 5, **dadurch gekennzeichnet, daß** die Biotinylierung der Zelloberfläche bei einer Temperatur von weniger als 10°C durchgeführt wurde.

## Revendications

1. Procédé de sélection de cellules sécrétant un premier anticorps, comprenant les étapes consistant à :
a) lier de l'avidine à la surface d'une cellule
b) incuber la cellule avec un polypeptide biotinylé, comprenant au moins un domaine fonctionnel de liaison à un anticorps provenant de la protéine A, de la protéine G, de la protéine L ou d' un variant fonctionnel de celui-ci
c) incuber la cellule avec un deuxième anticorps ou un antigène, qui est marqué par fluorescence et qui reconnaît un épitope sur le premier anticorps et se lie à celui-ci
d) sélectionner les cellules marquées par fluorescence qui ont lié une quantité du deuxième anticorps, au moyen d'une cytométrie de flux par analyse fluorescente.

2. Procédé selon la revendication 1, **caractérisé en ce que** le polypeptide biotinylé est une protéine G ou une protéine A.

3. Procédé selon la revendication 1, **caractérisé en ce que** l'avidine est liée à la surface cellulaire après qu'une biotinylation covalente de la surface cellulaire ait été effectuée.

4. Procédé selon la revendication 1, **caractérisé en ce que**, lors de l'incubation avec le polypeptide biotinylé ou au plus tard après celle-ci, les cellules sont cultivées dans un milieu comprenant un agent d'augmentation de la viscosité.

5. Procédé de sélection de cellules sécrétant un premier anticorps, comprenant les étapes consistant à :
1) lier de l'avidine à la surface d'une cellule, dans lequel l'avidine est conjuguée à un polypeptide comprenant au moins un domaine fonctionnel de liaison à un anticorps provenant de la protéine G ou de la protéine A.
2) incuber la cellule avec un deuxième anticorps ou un antigène, qui est marqué par fluorescence et qui reconnaît un épitope sur le premier anticorps et se lie à celui-ci
3) sélectionner les cellules marquées par fluorescence qui ont lié une quantité du deuxième anticorps, au moyen d'une cytométrie de flux par analyse fluorescente.

6. Procédé selon la revendication 5, **caractérisé en ce que** l'avidine est liée à la surface d'une cellule après qu'une biotinylation covalente de la surface cellulaire ait été effectuée.

7. Procédé selon l'une quelconque des revendications précédentes, **caractérisé en ce que** la cellule est une cellule sécrétant des anticorps.

8. Procédé selon la revendication 7, **caractérisé en ce que** la cellule est une cellule d'hybridome, de myélome ou de CHO.

9. Procédé selon l'une quelconque des revendications précédentes, **caractérisé en ce que** l'anticorps comprend des sites de liaison pour lier, avec une affinité élevée, une protéine G ou A bactérienne.

10. Procédé selon l'une quelconque des revendications précédentes, **caractérisé en ce que** l'anticorps est une IgG ou qu'il comprend une partie Fc d'une IgG.

11. Procédé selon l'une quelconque des revendications précédentes, **caractérisé en ce que** l'avidine est de la neutravidine.

12. Procédé selon la revendication 1, **caractérisé en ce que** la fraction de biotine est liée au polypeptide au moyen d'une fraction d'espacement, laquelle fraction d'espacement a une longueur d'au moins 15 Å.

13. Procédé selon la revendication 1 ou la 12, **caractérisé en ce que** le polypeptide biotinylé porte au moins 3 fractions de biotine, liées au polypeptide de manière covalente.

14. Procédé selon la revendication 13, **caractérisé en ce que** le polypeptide biotinylé comprend de 6 à 10 fractions de biotine.

15. Procédé selon la revendication 3 ou la 5, **caractérisé en ce qu'**une biotinylation de la surface de la cellule a été effectuée à une température inférieure à 10°C.
